# EUROPÄISCHE PATENTSCHRIFT

(11) **EP 2 241 350 B1**
(45) Veröffentlichungstag und Bekanntmachung des Hinweises auf die Patenterteilung: **30.05.2012**
(21) Anmeldenummer: 10155298.2
(22) Anmeldetag: 03.03.2010
(51) Int. Cl.: G21K 1/10

(54) **Anordnung zur Aufweitung der Partikelenergieverteilung eines Partikelstrahls, Partikeltherapieanlage sowie Verfahren zur Aufweitung der Partikelenergieverteilung eines Partikelstrahls**
Assembly for expanding the particle energy distribution of a particle beam, particle therapy assembly and method for expanding the particle energy distribution of a particle beam
Agencement d'élargissement de la répartition énergétique des particules d'un faisceau de particules, installation de traitement à particules ainsi que procédé d'élargissement de la répartition énergétique des particules d'un faisceau de particules

(30) Priorität: 15.04.2009 DE 102009017440
(43) Veröffentlichungstag der Anmeldung: 20.10.2010
(73) Patentinhaber: SIEMENS AKTIENGESELLSCHAFT, 80333 München (DE)
(72) Erfinder: Bönig, Marc-Oliver, 90419, Nürnberg (DE); Hein, Gerd, 91074, Herzogenaurach (DE)

(56) Entgegenhaltungen:
- TAKADA Y, KOBAYASHI Y, YASUOKA K, TERUNUMA T: "A miniature ripple filter for filtering a ripple found in the distal part of a proton SOBP" NUCLEAR INSTRUMENTS AND METHODS IN PHYSICS RESEARCH A, Bd. 524, 21. Mai 2004 (2004-05-21), Seiten 366-373, XP002585052
- SAKAE TAKEJI ET AL: "Multi-layer energy filter for realizing conformal irradiation in charged particle therapy" MEDICAL PHYSICS, AIP, MELVILLE, NY, US LNKD- DOI:10.1118/1.598840, Bd. 27, Nr. 2, 1. Februar 2000 (2000-02-01), Seiten 368-373, XP012011057 ISSN: 0094-2405

## Beschreibung

Anordnung zur Aufweitung der Partikelenergieverteilung eines Partikelstrahls, Partikeltherapieanlage sowie Verfahren zur Aufweitung der Partikelenergieverteilung eines Partikelstrahls

Die Erfindung betrifft eine Anordnung sowie ein Verfahren zur Aufweitung der Partikelenergieverteilung eines Partikelstrahls. Die Erfindung betrifft weiterhin eine Partikeltherapieanlage mit einer solchen Anordnung.

Bei einer Partikeltherapie, insbesondere von Krebserkrankungen, wird in einem geeigneten Beschleuniger ein Partikelstrahl beispielsweise aus Protonen oder Schwerionen, wie z.B. Kohlenstoffionen, erzeugt. Der Partikelstrahl wird in einem Beschleuniger erzeugt und in einen Behandlungsraum geführt und tritt dort über ein Austrittsfenster ein. In einer besonderen Ausführung kann der Partikelstrahl von einem Beschleuniger abwechselnd in verschiedene Behandlungsräume gelenkt werden. In dem Bestrahlungsraum ist ein zu therapierender Patient z.B. auf einem Patiententisch positioniert und gegebenenfalls immobilisiert.

Die Bestrahlung des Zielgebiets im Körper des Patienten, gewöhnlich ein Tumor, erfolgt in der Regel schichtweise. In Abhängigkeit von seiner Energie reicht der Partikelstrahl unterschiedlich tief in das Gewebe, so dass das Gewebe sozusagen in scheibenförmige Abschnitte oder Schichten gleicher Eindringtiefe unterteilt werden kann. Der fokussierte Partikelstrahl wird dann über die einzelnen Schichten des Zielgebiets bewegt, was als "Beam Scanning" bezeichnet wird, so dass innerhalb einer Schicht mehrere Punkte bestrahlt werden, die z.B. auf einem Rastergitter liegen. Indem die Strahlungsintensität bzw. die Energien richtig ausgewählt werden, können auch kompliziert geformte Bereiche genau bestrahlt werden. Die Anordnung der zu bestrahlenden Schichten und Punkte wird so gewählt, dass sich die geplante Dosisverteilung erzielen lässt.

Die Energie, die die Teilchen besitzen, wird durch den Beschleuniger erreicht, wobei der Partikelstrahl nahezu monoenergetisch ist. Die monoenergetischen Partikel deponieren dabei ihre Energie innerhalb eines sehr kleinen örtlichen Bereichs entlang der Strahlungsrichtung, innerhalb des so genannten "Bragg Peaks".

In der Partikeltherapie soll zumeist eine räumliche homogene Dosisverteilung erreicht werden, was eine räumliche Aufweitung der Energieverteilung des Partikelstrahls erfordert. Dabei ist ein gaußförmiges Dosistiefenprofil anstatt des Bragg Peaks entlang der Strahlachse erstrebenswert. Hierzu wird ein passives Filterelement in den Strahlengang zwischen dem Austrittsfenster und dem Patienten eingebracht, ein so genannter Ripplefilter, mit dessen Hilfe die gewünschte Aufweitung erzielt wird. Um Nebeneffekte zu minimieren, werden wasserähnliche Materialien als Material für das Filterelement verwendet. In den meisten Fällen werden Plexiglasplatten verwendet, in welche durch einen Fräsvorgang Rillen mit einer besonderen Geometrie eingebracht werden. Ein solcher Ripplefilter ist z.B. in dem Artikel "Design and construction of a ripple filter for a smoothed depth dose distribution in conformal particle therapy" von U. Weber und G. Kraft, Phys. Med. Biol. 44 (1999), 2765-2775, beschrieben. Ein Ripplefilter ist weiterhin beschrieben in dem Artikel "A miniature ripple filter for filtering a ripple found in the distal part of a proton SOBP, Takada Y, Kobayashi Y, Yasuoka K, Terunuma T, Nuclear Instruments and Methods in Physics Research A, Bd. 524, 21. Mai 2004, Seiten 366-373.

Für verschiedene Strahlaufweitungen werden verschiedene Geometrien bzw. Fräskonturen der Rillen benötigt. Durch die notwendige Genauigkeit der Rillengeometrie im Mikrometerbereich und bei einer Filtergröße von typischerweise 20 x 20cm ist die technische Machbarkeit in Bezug auf Genauigkeit und die Reproduzierbarkeit bei der Ausbildung der Rillen schwierig. Dies gilt vor allem für Filter für höhere gewünschte Strahlaufweitungen, da die Anforderungen an die Geometrie der Rillen (Verhältnis Rillenhöhe zu Rillenbreite) immer extremer werden. Hauptproblem ist dabei, dass es beim Fräsprozess des relativ weichen Plexiglases zu unerwünschten Wechselwirkungen zwischen Fräswerkzeug und Material kommt, wie Kompressionen und Verdrängung, und somit die gewünschte Genauigkeit und Reproduzierbarkeit nur schwer erreicht werden. Durch fräsen werden Ripplefilter für eine Strahlaufweitung von 2mm, 3mm und 4mm hergestellt, weiterhin einfach 2mm-, 3mm- und 4-mm Ripplefilter genannt. Dies bedeutet, dass anstelle des Bragg Peaks nach dem Filter sich ein gaußförmiges Profil des Strahles mit einer Halbwertsbreite von entsprechend 2mm, 3mm oder 4mm einstellt. Dabei hat es sich gezeigt, dass sich der 2mm-Ripplefilter mit der gewünschten Genauigkeit und Reproduzierbarkeit fertigen lässt, aber der 3mm-Ripplefilter und vor allem der 4mm-Ripplefilter viel schwieriger herstellbar sind.

Das Problem der erforderlichen hochgenauen Herstellung der Ripplefilter wurde bislang durch Optimierung von Fräser und Fräsverfahren gelöst, wobei dies mit hohen Herstellungskosten des Fräsers verbunden ist.

Der Erfindung liegt die Aufgabe zugrunde, eine Aufweitung des Partikelstrahls kostengünstig und mit einer hohen Präzision zu ermöglichen.

Die Aufgabe wird erfindungsgemäß gelöst durch eine Anordnung zur Aufweitung der Partikelenergieverteilung eines Partikelstrahls, umfassend zwei Ripplefilter, die in Strahlungsrichtung in Reihe hintereinander angeordnet sind.

Die Erfindung basiert auf der Überlegung, dass ein technisch schwer herstellbarer Filter mit gewünschter hoher Strahlaufweitung mittels zweier oder mehrerer leicht herstellbarer Filter niedriger Strahlaufweitung realisierbar ist. Dabei werden die zwei oder mehreren Ripplefilter in Strahlungsrichtung in Reihe hintereinander angeordnet, um die Partikelenergieverteilung stufenweise zu modifizieren.

Durch die mehrfache Anordnung von z.B. 2mm- und 3mm-Ripplefiltern können Filter mit höherer Strahlaufweitung, z.B. 4mm oder 5mm, realisiert werden. Dabei führt die größere Menge an Material, das in den Strahlengang geführt wird, zu einem erhöhten Energieverlust, der allerdings konstant ist und einfach korrigiert werden kann, indem die Partikeltherapie mit einer höheren Strahlenergie durchgeführt wird.

Bei einer Reihenschaltung zweier Filter, die gleich ausgerichtet sind, sollen die Rillen der beiden Filter exakt zueinander ausgerichtet sein. Eine deutliche Vereinfachung der Anordnung der einzelnen Ripplefilter wird gewährleistet, indem die Ripplefilter vorzugsweise zueinander verdreht sind. Durch die Verdrehung der Ripplefilter zueinander ist auch die Kombination mehrerer hintereinander positionierter Filter ermöglicht.

Bevorzugt sind zwei Ripplefilter vorgesehen, die um 90° zueinander verdreht sind. Dabei werden Fertigungstoleranzen der einzelnen Filter statistisch ausgeglichen, da die Rillenstrukturen beider Ripplefilter orthogonal überlagert sind.

Gemäß einer bevorzugten Ausgestaltung sind die verwendeten Ripplefilter für eine Strahlaufweitung von 2mm und/oder von 3mm ausgebildet. Mit den herkömmlichen Herstellungsverfahren lassen sich solche Ripplefilter mit hoher Präzision und Reproduzierbarkeit fertigen, so dass durch die Hintereinanderschaltung dieser Filter eine größere Aufweitung des Partikelstrahls mit geringem Aufwand erreicht wird.

Nach einer bevorzugten Variante sind die verwendeten zwei Ripplefilter ein Filter für eine Strahlaufweitung von 2mm und ein nachgeschalteter Filter für eine Strahlaufweitung von 3mm. Durch diese Anordnung, die besonders einfach zu konstruieren und reproduzieren ist, wird eine Strahlaufweitung von 4mm erreicht.

Vorteilhafterweise sind die zwei Ripplefilter in einem Abstand von maximal etwa 1m hintereinander angeordnet. Dies bedeutet, dass der Ripplefilter für die 3mm-Aufweitung maximal etwa 1m hinter dem Filter für die 2mm-Aufweitung positioniert ist. Um den Raumbedarf für die Anordnung zu reduzieren, ist es wünschenswert, einen möglichst geringen Abstand zwischen den beiden Filtern einzustellen, insbesondere die beiden Filter aufeinander zu kleben.

Vorzugsweise sind die Ripplefilter nach Art von Platten mit einer periodischen Struktur aus feinen Rillen gebildet. Da bei der beschriebenen Anordnung Ripplefilter für eine Aufweitung von 2mm oder 3mm eingesetzt werden, bereitet die Bearbeitung der Platten zum Ausbilden der feinen Rillen keinen so großen Aufwand. Es kann dabei die gewünschte Form der Rillen mit einer mechanischen Präzision von etwa 5µm bis 10µm produziert werden.

Zweckdienlicherweise sind die Ripplefilter aus Polymethylmethacrylat (PMMA).

Die Aufgabe wird weiterhin erfindungsgemäß gelöst durch eine Partikeltherapieanlage mit einer Anordnung nach einer der vorhergehenden Ausführungen.

Die Aufgabe wird zudem erfindungsgemäß gelöst durch ein Verfahren zur Aufweitung der Partikelenergieverteilung eines Partikelstrahls, bei dem mindestens zwei Ripplefilter in Strahlungsrichtung in Reihe hintereinander geschaltet werden. Die in Bezug auf die Anordnung aufgeführten Vorteile und bevorzugten Ausgestaltungen sind sinngemäß auf das Verfahren zu übertragen.

Ein Ausführungsbeispiel der Erfindung wird im Folgenden anhand einer Zeichnung erläutert. Hierin zeigen:
- FIG 1: eine stark vereinfachte schematische Darstellung einer Partikeltherapieanlage,
- FIG 2: in einer perspektivischen Darstellung den Aufbau eines Ripplefilters,
- FIG 3: schematisch zwei Ripplefilter, die in Strahlungsrichtung hintereinander angeordnet sind, und
- FIG 4: in einem Diagramm die Ergebnisse einer Messung mit der Anordnung gemäß FIG 3.

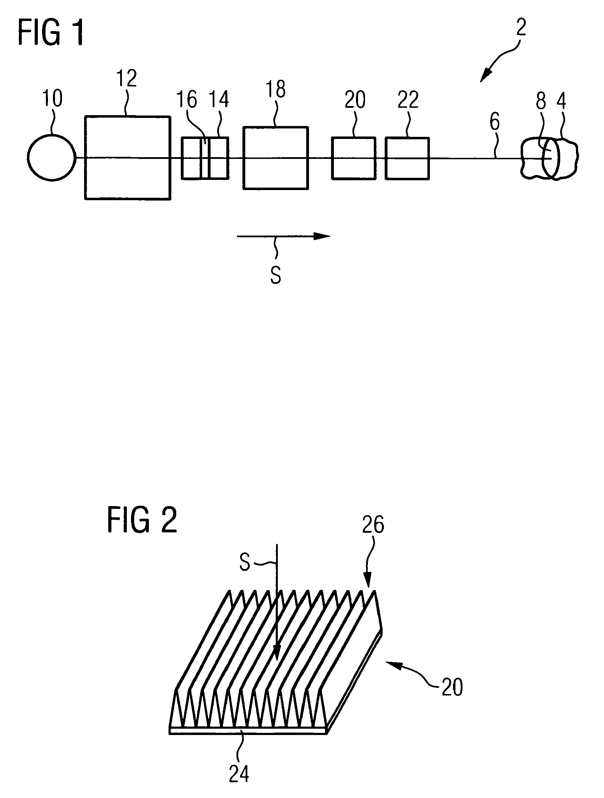

In FIG 1 ist eine Prinzipskizze einer Partikeltherapieanlage 2 gezeigt. Die Partikeltherapieanlage 2 wird eingesetzt, um ein Tumorgewebe 4 mit Hilfe eines Partikelstrahls 6 schrittweise zu bestrahlen, wobei bei jedem Schritt ein scheibenförmiger Abschnitt 8 des Tumors 4 behandelt wird. Der Partikelstrahl wird in einem Beschleuniger 10 generiert, der von einer Steuereinheit 12 angesteuert wird. Der Beschleuniger 10 liefert die Partikel mit einer Energie, die für die aktuell zu bestrahlende Schicht 8 erforderlich ist. Die Energie liegt typischerweise im zwei- bis dreistelligen MeV-Bereich, beispielsweise im Bereich um 100MeV. Die Steuereinheit 12 umfasst unter anderem eine hier nicht gezeigte Rasterscanvorrichtung, die den Strahl 6 sowohl in horizontaler als auch in vertikaler Richtung ablenkt, um das Tumorgewebe innerhalb der Schicht 8 abzutasten. Dazu umfasst die Rasterscanvorrichtung beispielsweise zwei Magnetpaare.

Bei der Bestrahlung des Tumors 4 eines nicht dargestellten Patienten wird über den Beschleuniger 10 ein hohes Energieniveau des Partikelstrahls eingestellt, so dass der Partikelstrahl 6 den in der Figur rechten Randbereich des Tumors 4 erreicht. Dabei werden mehrere Rasterpunkte der erreichten Schicht 8 des Tumors 4 punktuell bestrahlt.

Der Strahl 6 verläuft üblicherweise außerdem durch einen im Strahlengang angeordneten Energiemodulator 14 mit einem Absorberelement 16, welches einen Teil der Energie des Partikelstrahls absorbiert, wenn der Partikelstrahl 6 durch sein Material durchgeht, und somit die Reichweite der Partikel beschränkt. Anschließend tritt der Partikelstrahl 6 durch ein Kontrollsystem 18, das insbesondere nach Art eines Teilchenzählers ausgebildet ist. Die im Bereich des Tumors 4 deponierte Partikeldosis hängt von der im Strahl 6 vorhandenen Partikelzahl ab. Während des Bestrahlungsvorgangs wird mittels des Kontrollsystems 18 die auf den Tumor 4 einwirkende Partikelzahl ermittelt. Bei Erreichen der gewünschten Partikelzahl in einem Rasterpunkt wird ein Signal an die Steuereinrichtung 12 abgegeben, welche die Rasterscaneinrichtung dafür ansteuert, den Strahl auf den nächsten Rasterpunkt auszurichten.

In dem gezeigten Ausführungsbeispiel sind strahlabwärts außerdem Mittel zur Veränderung der räumlichen Energieverteilung des Partikelstrahls 6 vorgesehen. Die Mittel umfassen hierbei einen Ripplefilter 20 zur Aufweitung des Strahles 6 in Strahlungsrichtung S und eine Fokussiereinrichtung 22 zur Aufweitung des Strahles 6 radial zur Strahlungsrichtung S.

Der prinzipielle Aufbau eines Ripplefilters 20 für eine Partikelenergieaufweitung von 2mm ist in FIG 2 gezeigt. Der Ripplefilter 20 ist aus einer dünnen Plexiglasplatte 24 (PMMA) hergestellt, die beispielsweise etwa 200 x 200 x 2mm groß ist. Der gezeigte Ripplefilter 20 weist eine periodische Struktur von mehreren feinen und genau gefrästen Rillen 26 mit hochgenauer Querschnittsgeometrie auf.

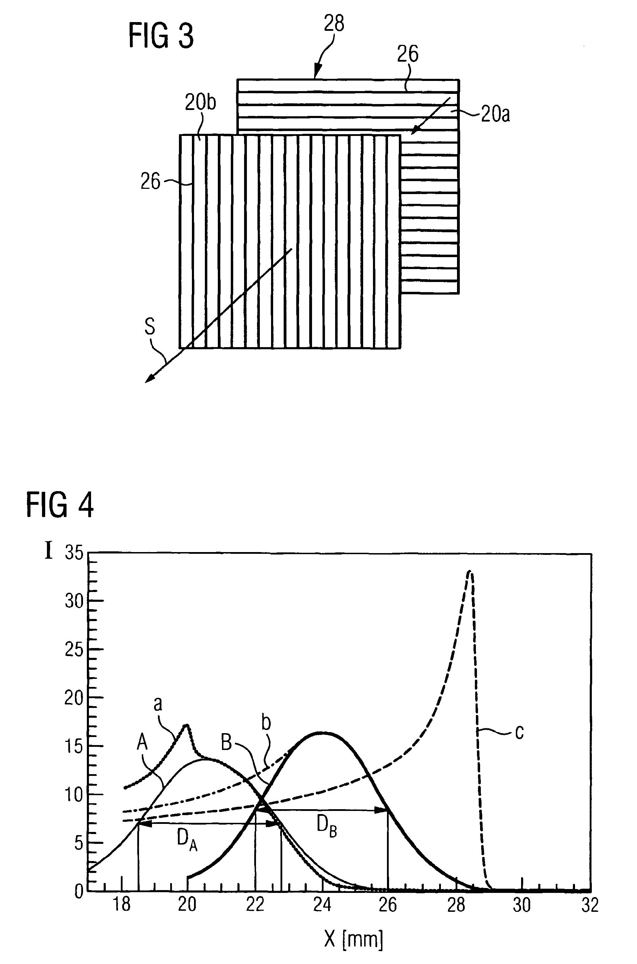

Um eine Aufweitung des Partikelstrahls 6 beispielsweise von 4mm zu erreichen, ist in Strahlungsrichtung S eine Anordnung 28 von zwei hintereinander positionierten Ripplefiltern 20a, 20b eingebracht, wie aus FIG 3 ersichtlich. Der erste Ripplefilter 20a in Strahlungsrichtung ist ein Filter zur Aufweitung des Partikelstrahls 6 von 2mm und der nachgeschaltete Filter 20b weitet den Partikelstrahl 6 um 3mm auf. Die Ripplefilter 20a, 20b sind etwa 1m auseinander positioniert. Außerdem sind die Filter 20a, 20b um 90° zueinander verdreht, so dass die Rillen 26 beider Filter 20a, 20b orthogonal zueinander verlaufen.

Mit Hilfe einer in FIG 3 gezeigten Anordnung 28 wurde im Rahmen eines Versuchs die Aufweitung der Partikelenergieverteilung gemessen und die Ergebnisse dieser Messung ist in FIG 4 graphisch dargestellt ist. Im Diagramm in FIG 4 ist die relative Dosis I über der Eindringtiefe X des Strahles 6 in Millimetern aufgetragen. Die Messung mit der Anordnung 28 ist durch die Kurve "b" angegeben, welche einen gaußförmigen Verlauf aufweist. Die Messung mit beiden hintereinander geschalteten Filtern 20a, 20b wurde bei einer Strahlenergie von 108,53 MeV durchgeführt. Diese erhöhte Strahlenergie wurde gewählt, um den zusätzlichen Energieverlust durch das zweite Filterelement zu kompensieren.

Als Vergleich ist eine Messung an einem 4mm-Ripplefilter eingetragen, die bei einer Energie von 88,83 MeV durchgeführt war. Im Diagramm in FIG 4 ist diese Kurve mit "a" gekennzeichnet.

Zusätzlich wurde eine Messung ohne Filterelemente als Referenzmessung durchgeführt. Die Referenzmessung zeigt den ungestörten Bragg Peak, welcher mit "c" gekennzeichnet ist.

An die beiden Kurven a und b der Messungen mit dem 4mm-Ripplefilter und mit der Kombination von einem 2mm- mit einem 3mm-Ripplefilter ist jeweils eine Gauß-Funktion angepasst, die durch die Kurven A bzw. B dargestellt ist.

Die Aufweitung des Bragg Peaks bei der Verwendung zweier um 90° verdrehter Ripplefilter von 2mm und 3mm ergibt eine Aufweitung, die im Wesentlichen mit der eines einzelnen 4mm-Ripplefilter vergleichbar groß ist. Dies ist anhand der Halbwertsbreiten (FWHM) der Kurven A und B erkennbar: die Halbwertsbreite D_{A} der Kurve A bei Verwendung des 4mm-Filters entspricht etwa der Halbwertsbreite D_{B} der Kurve B bei Verwendung der Kombination des 2mm-Ripplefilters mit dem 3mm-Ripplefilter und liegt im Rahmen der Auswertegenauigkeit bei etwa 4mm. Bei "b" ist keine fehlerhafte Überhöhung wie bei der Kurve "a" zu erkennen. Dies zeigt experimentell, dass die Reihenschaltung toleranter gegenüber Fertigungsfehlern ist. Da die Filterelemente um 90° verdreht verwendet werden, gleichen sich die Fertigungsfehler beider Filter aus, so dass hier die Fertigungstoleranzen für die einzelnen Filterelemente größer sein können. Da durch die zwei Filterelemente eine insgesamt größere Gesamtdicke vorliegt, wurde die zusätzliche Dicke durch eine höhere Strahlenergie kompensiert.

### Bezugszeichenliste

- 2: Partikeltherapieanlage
- 4: Tumor
- 6: Partikelstrahl
- 8: Schicht
- 10: Beschleuniger
- 12: Steuereinrichtung
- 14: Energiemodulator
- 16: Absorberelement
- 18: Kontrollsystem
- 20,20a,20b: Ripplefilter
- 22: Fokussiereinrichtung
- 24: Plexiglasplatte
- 26: Rille
- 28: Anordnung

- A,B: Verlauf der Gauß-Funktionen
- a, b, c: Kurvenverlauf der Messungen
- D_{A}, D_{B}: Halbwertbreiten
- I: normierte Intensität
- S: Strahlungsrichtung
- X: Eindringtiefe

## Patentansprüche

1. Anordnung (28) zur Aufweitung der Partikelenergieverteilung eines Partikelstrahls (6), **gekennzeichnet durch** zwei Ripplefilter (20a, 20b), die in Strahlungsrichtung (S) in Reihe hintereinander angeordnet sind.

2. Anordnung (28) nach Anspruch 1,
wobei die Ripplefilter (20a, 20b) jeweils eine periodische Rillenstruktur aufweisen und die Rillenstrukturen zueinander verdreht sind.

3. Anordnung (28) nach Anspruch 2,
wobei zwei Ripplefilter (20a, 30b) vorgesehen sind, bei denen die Rillenstruktur um 90° zueinander verdreht sind.

4. Anordnung (28) nach einem der vorhergehenden Ansprüche,
wobei die Ripplefilter (20a, 20b) für eine Strahlaufweitung von 2mm und/oder von 3mm ausgebildet sind.

5. Anordnung (28) nach Anspruch 2 und 3,
wobei die zwei Ripplefilter (20a, 20b) ein Filter (01a) für eine Strahlaufweitung von 2mm und ein nachgeschalteter Filter (10b) für eine Strahlaufweitung von 3mm sind.

6. Anordnung (28) nach Anspruch 2, 4 oder 5,
wobei die zwei Ripplefilter (20a, 20b) in einem Abstand von maximal etwa 1m hintereinander angeordnet sind.

7. Anordnung (28) nach einem der vorhergehenden Ansprüche,
wobei die Ripplefilter (20a, 20b) nach Art von Platten (24) mit einer periodischen Struktur aus feinen Rillen (26) ausgebildet sind.

8. Anordnung (28) nach einem der vorhergehenden Ansprüche,
wobei die Ripplefilter (20a, 20b) aus Polymethylmethacrylat sind.

9. Partikeltherapieanlage (2) mit einer Anordnung (28) nach einem der vorhergehenden Ansprüche.

10. Verfahren zur Aufweitung der Partikelenergieverteilung eines Partikelstrahls (6), **gekennzeichnet dadurch, dass** zumindest zwei Ripplefilter (20a, 20b) in Strahlungsrichtung (S) in Reihe hintereinander geschaltet werden.

## Claims

1. Arrangement (28) for expanding the particle energy distribution of a particle beam (6), **characterised by** two ripple filters (20a, 20b) which are arranged in series one behind the other in the radiation direction (S).

2. Arrangement (28) according to claim 1,
wherein the ripple filters (20a, 20b) each have a periodic groove structure and the groove structures are rotated relative to each other.

3. Arrangement (28) according to claim 2,
wherein two ripple filters (20a, 30b) are provided in which the groove structures are rotated through 90° relative to each other.

4. Arrangement (28) according to one of the preceding claims, wherein the ripple filters (20a, 20b) are embodied for a beam expansion of 2mm and/or 3mm.

5. Arrangement (28) according to claim 2 and 3,
wherein the two ripple filters (20a, 20b) are a filter (01a) for a beam expansion of 2mm and a filter (10b) connected downstream thereof for a beam expansion of 3mm.

6. Arrangement (28) according to claim 2, 4 or 5,
wherein the two ripple filters (20a, 20b) are arranged one behind the other at a maximum spacing of about 1m apart.

7. Arrangement (28) according to one of the preceding claims, wherein the ripple filters (20a, 20b) are embodied in the manner of plates (24) having a periodic structure consisting of fine grooves (26).

8. Arrangement (28) according to one of the preceding claims, wherein the ripple filters (20a, 20b) are made of polymethylmethacrylate.

9. Particle therapy system (2) having an arrangement (28) according to one of the preceding claims.

10. Method for expanding the particle energy distribution of a particle beam (6), **characterised in that** at least two ripple filters (20a, 20b) are connected in series one behind the other in the radiation direction (S).

## Revendications

1. Agencement (28) pour l'élargissement de la répartition énergétique de particules d'un faisceau de particules (6), **caractérisé par** deux "filtres Ripple" (20a, 20b), qui sont disposés en ligne l'un derrière l'autre dans le sens de rayonnement (S).

2. Agencement (28) selon la revendication 1,
les "filtres Ripple" (20a, 20b) présentant à chaque fois une structure rainurée périodique et les structures de rainure étant tournées les unes par rapport aux autres.

3. Agencement (28) selon la revendication 2,
deux "filtres Ripple" (20a, 30b) étant prévus, sur lesquels les structures rainurées sont tournées de 90° l'une par rapport à l'autre.

4. Agencement (28) selon l'une des revendications précédentes,
les "filtres Ripple" (20a, 20b) étant conçus pour un élargissement de faisceau de 2 mm et/ou de 3 mm.

5. Agencement (28) selon les revendications 2 et 3,
les deux "filtres Ripple" (20a, 20b) étant un filtre (01a) pour un élargissement de faisceau de 2 mm et un filtre (10b) monté an aval pour un élargissement de faisceau de 3 mm.

6. Agencement (28) selon la revendication 2, 4 ou 5,
les deux "filtres Ripple" (20a, 20b) étant disposés l'un derrière l'autre à un espacement maximum d'environ 1 m.

7. Agencement (28) selon l'une des revendications précédentes,
les "filtres Ripple" (20a, 20b) étant conçus à la façon de plaques (24) avec une structure périodique à base de rainures (26) fines.

8. Agencement (28) selon l'une des revendications précédentes,
les "filtres Ripple" (20a, 20b) étant à base de polyméthylméthacrylate.

9. Installation de thérapie à particules (2) comprenant un agencement (28) selon l'une des revendications précédentes.

10. Procédé pour l'élargissement de la répartition énergétique de particules d'un faisceau de particules (6), **caractérisé en ce qu'**au moins deux "filtres Ripple" (20a, 20b) sont montés en série l'un derrière l'autre dans le sens de rayonnement (S).
